(19) Europäisches Patentamt
European Patent Office
Office européen des brevets

(11) **EP 1 864 712 B2**

(12) **NOUVEAU FASCICULE DE BREVET EUROPEEN**
Après la procédure d'opposition

(45) Date de publication et mention de la décision concernant l'opposition:
**29.01.2020 Bulletin 2020/05**

(45) Mention de la délivrance du brevet:
**05.06.2013 Bulletin 2013/23**

(21) Numéro de dépôt: **07115954.5**

(22) Date de dépôt: **16.02.2000**

(51) Int Cl.:
*B01J 20/18* (2006.01)  *C07C 7/13* (2006.01)
*C07C 15/08* (2006.01)  *C07C 15/02* (2006.01)
*C07C 37/82* (2006.01)  *C07H 1/06* (2006.01)
*C07C 29/76* (2006.01)  *C07C 201/16* (2006.01)
*C07C 209/86* (2006.01)  *C07C 211/50* (2006.01)

(54) **Procédé d'obtention d'adsorbants zéolitiques agglomerés et leurs utilisation**

Verfahren zur Herstellung von Adsorptionsmitteln aus agglomerierten Zeolithen und deren Verwendungen

Process of preparation of agglomerated zeolite adsorbents and uses thereof

(84) Etats contractants désignés:
**AT BE CH CY DE DK ES FI FR GB GR IE IT LI LU MC NL PT SE**

(30) Priorité: **22.02.1999 FR 9902151**

(43) Date de publication de la demande:
**12.12.2007 Bulletin 2007/50**

(62) Numéro(s) de document de la (des) demande(s) initiale(s) en application de l'article 76 CBE:
**00905148.3 / 1 159 065**

(73) Titulaires:
• **ARKEMA FRANCE**
**92700 Colombes (FR)**
• **IFP Energies nouvelles**
**92500 Rueil-Malmaison (FR)**

(72) Inventeurs:
• **Plee, Dominique**
**64140 Lons (FR)**
• **Méthiver, Alain**
**69004 Lyon (FR)**

(74) Mandataire: **Bandpay & Greuter**
**30, rue Notre-Dame des Victoires**
**75002 Paris (FR)**

(56) Documents cités:
EP-A- 0 115 068    EP-A- 0 137 063
EP-A- 0 531 191    EP-A- 0 893 157
FR-A- 2 766 475    FR-A- 2 767 524
FR-A1- 2 767 524   US-A- 3 119 660
US-A- 3 558 730    US-A- 3 960 774
US-A- 4 642 397    US-A- 5 149 887
US-A- 5 849 981    US-A- 5 849 981

• Breck Donald W.: "Zeolite molecular sieves", 1973, John Wiley & Sons Inc., USA pages 92, 93-176, 428, 429,
• Dubinin M.: Chem. Rev., vol. 60, 1960, pages 235-241,
• Dubinin M.: J. Colloid and Interface Sci., vol. 23, 1967, pages 481-499,
• Lecloux: Rapport de la société royale des sciences de Liège, 1971, pages 169-209,
• Détermination du volume microporeaux par la méthode de Dubinin.
• Rapport d'essais relatifs à l'échange des ioms baryum. (titulaires)
• Calculs relatifs à l'échange des ions baryum (titulaires)
• Clay mineralogy, 1968, Contents et p. 185 à 189

EP 1 864 712 B2

**Description**

DOMAINE TECHNIQUE

Le domaine de l'invention est celui des adsorbants zéolitiques agglomérés à base de zéolite X échangée au baryum.

TECHNIQUE ANTERIEURE

L'art antérieur a reconnu que les adsorbants constitués de zéolites X ou Y échangées au moyen d'ions tels que baryum, potassium ou strontium, seuls ou en mélange, sont efficaces pour adsorber sélectivement le paraxylène dans un mélange contenant au moins un autre isomère aromatique en $C_8$. Les brevets US 3.558.730, US 3.558.732, US 3.626.020 et US 3.663.638 divulguent des adsorbants comprenant des aluminosilicates échangés par du baryum et du potassium qui séparent efficacement le paraxylène d'un mélange d'isomères aromatiques en $C_8$.

US 3.878.127 décrit une méthode de préparation d'adsorbants destinés à la séparation des xylènes qui consiste à traiter dans la soude à chaud des agglomérés (zéolite X +liant) de rapport $Na_2O/Al_2O_3$ strictement inférieur à 0,7 afin de remplacer les cations échangeables de la zéolite (tels que protons ou cations du Groupe IIA) par du sodium préalablement à un échange baryum ou baryum+potassium, l'échange préalable au sodium permettant à une plus grande quantité d'ions baryum ou baryum+ potassium d'être ajoutés à la structure zéolitique.

Ces adsorbants sont utilisés comme agents d'adsorption dans les procédés en phase liquide, de préférence de type contre-courant simulé similaires à ceux décrits dans US 2,985,589, qui s'appliquent entre autres aux coupes de $C_8$ aromatiques issues, par exemple, des procédés de dialkylation du benzène, dans les procédés en phase gazeuse.

Les zéolites X échangées au baryum ont de nombreuses autres applications en tant qu'agents d'adsorption parmi lesquelles on peut citer :

    * la séparation de sucres, voir par exemple EP 115.631, EP 115.068,
    * la séparation d'alcools polyhydriques (EP 137.063),
    * la séparation d'isomères de toluène substitué US 4.642.397 (nitrotoluène), US 4.940.548 (diéthyltoluène), US 4.633.018 (toluènediamine)
    * la séparation des crésols (US 5.149.887).

Dans les références listées ci-dessus, les adsorbants zéolitiques se présentent sous forme de poudre ou sous forme d'agglomérés constitués majoritairement de zéolite et d'au moins 15 à 20 % en poids de liant inerte et dont le volume de Dubinin mesuré par adsorption d'azote à 77 °K après dégazage sous vide à 300 °C pendant 16 heures est inférieur à 0,230 cm$^3$/g.

La synthèse des zéolites X s'effectuant principalement par nucléation et cristallisation de gels de silicoaluminates, on obtient des poudres dont l'emploi à l'échelle industrielle est particulièrement malaisé (pertes de charges importantes lors des manipulations des poudres) et l'on préfère les formes agglomérées granulaires. Ces agglomérés, qu'ils soient sous forme de plaquettes, de billes ou d'extrudés, sont couramment constitués d'une poudre de zéolite, qui constitue l'élément actif et d'un liant destiné à assurer la cohésion des cristaux sous forme de grains. Ce liant n'a aucune propriété adsorbante, sa fonction étant de conférer au grain une résistance mécanique suffisante pour résister aux vibrations et aux mouvements auxquels il est soumis au cours de ses divers emplois. Les agglomérés sont préparés par empâtage de poudre de zéolite avec une pâte argileuse, dans des proportions de l'ordre de 80 à 85 % de poudre de zéolite pour 20 à 15 % de liant, puis mise en forme en billes, plaquettes ou extrudés, et traitement thermique à haute température pour cuisson de l'argile et réactivation de la zéolite, l'échange au baryum pouvant être effectué soit avant soit après l'agglomération de la zéolite pulvérulente avec le liant. Le résultat en est des corps zéolitiques dont la granulométrie est de quelques millimètres, et qui, si le choix du liant et la granulation sont faits dans les règles de l'art, présentent un ensemble de propriétés satisfaisantes, en particulier de porosité, de résistance mécanique, de résistance à l'abrasion. Cependant, les propriétés d'adsorption sont évidemment réduites dans le rapport de la poudre active à la poudre et son liant inerte d'agglomération.

Divers moyens ont été proposés pour pallier cet inconvénient du liant d'être inerte quant aux performances adsorbantes, parmi lesquels, la transformation du liant, pour tout ou partie, en zéolite. Cette opération s'effectue facilement lorsqu'on utilise des liants de la famille de la kaolinite, préalablement calcinés à des températures comprises entre 500°C et 700°C. Une variante consiste à mouler des grains de kaolin et à les zéolitiser : son principe est exposé dans "ZEOLITE MOLECULAR SIEVES" de D.W. BRECK, John Wiley and Sons, NEW YORK. Cette technologie a été appliquée avec succès à l'obtention de grains de zéolite A ou X, constitués jusqu'à 95 % en poids de la zéolite elle-même et d'un résiduel de liant non transformé (voir à cet effet US 3.119.660), l'ajout d'une source de silice étant recommandé lorsque l'on veut obtenir une zéolite X ("ZEOLITE MOLECULAR SIEVES", BRECK, p. 320).

FLANK et collaborateurs montrent dans US 4.818.508 que l'on peut préparer des agglomérés à base de zéolite A, X ou Y par digestion de préformes d'argile réactive (obtenue par traitement thermique d'argile non-réactive -telle que halloysite ou kaolinite- dont au moins 50 % en poids se présente sous forme de particules de granulométrie comprise entre 1,5 et 15 μm de préférence en présence d'agent porogène) avec un oxyde de métal alcalin. Les exemples relatifs à la synthèse d'agglomérés à base de zéolite X montrent qu'il est nécessaire d'ajouter une source de silice, ce qui n'est pas le cas pour préparer des agglomérés à base de zéolite A.

JP-05163015 (Tosoh Corp.) enseigne que l'on peut former des grains de zéolite X à rapport Si/Al faible, inférieur à 1,25, en mélangeant une poudre de zéolite LSX de rapport Si/Al = 1,25 avec du kaolin, de la potasse, de la soude et de la carboxyméthylcellulose. On met en forme par extrusion. Les grains ainsi obtenus sont séchés, calcinés à 600°C pendant 2 heures puis immergés dans une solution de soude et de potasse à 40°C pendant 2 jours.

Ces deux documents enseignent que l'on peut préparer des solides résistants mécaniquement. Néanmoins, les procédés associés sont lourds et pèchent, soit par la durée excessive de réaction, soit par le nombre d'étapes mises en jeu. On peut craindre, d'autre part, que le traitement thermique tel que revendiqué dans JP 05-163015, après l'étape de mise en forme, ne contribue à l'amorphisation du grain et que la digestion caustique qui suit ait pour objet de le recristalliser, ce qui expliquerait la lenteur du procédé.

La présente invention a pour objet un procédé d'obtention d'un adsorbant zéolitique aggloméré consistant en les étapes suivantes :

a/ on agglomère une zéolite NaX industrielle, de rapport Si/Al = 1,25 et de rapport Na/Al = 1, en mélangeant intimement 800 g de poudre de zéolite X, (exprimés en équivalent calciné), 150 g de kaolin (exprimés en équivalent calciné), 56 g de silice colloïdale (et contenant 30 % en poids de $SiO_2$ et 0,5 % de $Na_2O$) et 6 g de carboxyméthylcellulose avec la quantité d'eau adéquate pour l'extrusion ; l'extrudé est séché, concassé de manière à récupérer des grains dont le diamètre équivalent est égal à 0,7 mm, puis calciné à 550 ° C sous courant d'azote pendant 2 h ;

b/200 g de granulés ainsi obtenus sont placés dans un réacteur en verre muni d'une double enveloppe régulée à une température de 100±1 °C puis on ajoute 1,5 l d'une solution aqueuse de soude de concentration 100 g/l et laisse le milieu réactionnel sous agitation pendant 3 h ; on procède ensuite au lavage des granulés en 3 opérations successives de lavage à l'eau suivi de la vidange du réacteur ; on s'assure de l'efficacité du lavage en mesurant le pH final des eaux de lavage qui doit être compris entre 10 et 10,5 ;

c/ on procède ensuite à un échange baryum au moyen d'une solution de chlorure de baryum 0,6 M à 95°C en 4 étapes ; à chaque étape, le rapport du volume de solution sur la masse de solide est de 20 ml/g et l'échange est poursuivi pendant 4 heures à chaque fois ; suivi d'un lavage puis d'un séchage à 80 °C pendant 2 h entre chaque échange, le solide est lavé plusieurs fois de manière à le débarrasser des excédents de sel ;

d/ il est ensuite activé à une température de 250 °C pendant 2 h sous courant d'azote.

L'invention concerne également les utilisations de l'adsorbant zéolitique issu du procédé decrit ci-dessus comme agent d'adsorption susceptible de remplacer avantageusement les agents d'adsorption décrits dans la littérature à base de zéolite X échangée au baryum et notamment dans les utilisations listées ci-dessous :

* la séparation des isomères aromatiques en $C_8$ et notamment des xylènes,
* la séparation de sucres,
* la séparation d'alcools polyhydriques,
* la séparation d'isomères de toluène substitué tels que nitrotoluène, diéthyltoluène, toluènediamine,
* la séparation des crésols.

L'invention concerne notamment un perfectionnement de procédé de récupération de paraxylène à partir de coupes d'isomères $C_8$ aromatiques consistant à utiliser comme agent d'adsorption du p-xylène un adsorbant zéolitique susceptible d'être obtenu par le procédé selon l'invention, mis en oeuvre dans de procédés en phase liquide mais aussi en phase gazeuse.

On peut ainsi séparer le produit désiré par chromatographie liquide d'adsorption préparative (en batch), avantageusement en lit mobile simulé, c'est-à-dire à contre-courant simulé ou à co-courant simulé, et plus particulièrement à contre-courant simulé.

Les conditions opératoires d'une unité industrielle d'adsorption de type contre-courant simulé sont en général les suivantes :

| | |
|---|---|
| nombre de lits | 6 à 30 |
| nombre de zones | au moins 4 |
| température | 100 à 250°C, |

(suite)

| | |
|---|---|
| de préférence | 150 à 190 °C |
| pression | 0,2 à 3 MPa |
| rapport des débits désorbant sur charge | 1 à 2,5 |
| (par exemple 1,4 à 1,8 pour une unité d'adsorption seule (stand alone) et 1,1 à 1,4 pour une unité d'adsorption combinée à une unité de cristallisation) | |
| taux de recyclage | 3,5 à 12, |
| de préférence | 4 à 6 |

On pourra se référer aux brevets US 2.985.589, US 5.284.992 et US 5.629.467.

Les conditions opératoires d'une unité industrielle d'adsorption à co-courant simulé sont en général les mêmes que celles fonctionnant à contre-courant simulé à l'exception du taux de recyclage qui est en général compris entre 0,8 et 7. On pourra se référer aux brevets US 4.402.832 et US 4.498.991.

Le solvant de désorption peut être un désorbant dont le point d'ébullition est inférieur à celui de la charge, tel que le toluène mais aussi un désorbant dont le point d'ébullition est supérieur à celui de la charge, tel que le paradiéthylbenzène (PDEB)

La sélectivité des adsorbants selon l'invention pour l'adsorption du p-xylène contenu dans des coupes aromatiques en $C_8$ est optimale lorsque leur perte au feu mesurée à 900 °C est comprise en général entre 4,0 et 7,7 %, et de préférence entre 5,2 et 7,7 %. De l'eau et un peu de dioxyde de carbone entrent dans la perte au feu.

Les exemples suivants illustrent l'invention.

EXEMPLES

Ces exemples font appel à la mesure ou l'appréciation de certaines grandeurs caractéristiques des adsorbants de l'invention.

Pour apprécier la sélectivité qu'offre l'adsorbant d'un procédé de séparation du paraxylène, on lui applique un test qui permet la mesure de son pouvoir séparateur entre le paraxylène (PX) et ses isomères $C_8$ aromatiques (MX, OX), mais aussi entre paraxylène et éthylbenzène (EB), ce qui est important parce que certaines coupes peuvent être riches en éthylbenzène et ne pas l'être en autres isomères $C_8$, et également entre le paraxylène et le désorbant, parce qu'il est tout aussi important de disposer d'une sélectivité faible PX / désorbant, condition pour que la désorption soit efficace.

Le test consiste à immerger un adsorbant (17 g) préalablement activé thermiquement et refroidi à l'abri de l'air, dans 80 g d'un mélange d'aromatiques dissous dans du 2,2,4-triméthylpentane.

La composition exacte du mélange est la suivante :

| | |
|---|---|
| PX | 2% |
| MX | 2% |
| OX | 2% |
| EB | 2 % |
| toluène (désorbant) | 2 % |
| 2,2,4-triméthylpentane | le reste |

On procède à l'autoclave à 150°C, pendant 4 heures, durée suffisante pour assurer l'équilibre d'adsorption. Une partie du liquide est alors prélevée, condensée à -30°C et analysée par chromatographie en phase gazeuse. Il est alors possible de remonter aux concentrations dans la phase adsorbée et dans la phase non adsorbée et d'exprimer la quantité de paraxylène adsorbée et les sélectivités en paraxylène par rapport aux autres aromatiques et au désorbant. Le 2,2,4-triméthylpentane ne perturbe pas ces résultats, étant très peu adsorbé. Pour les exemples 1 et 2 ci-dessous, le désorbant mis en oeuvre est le toluène.

On mesure la sélectivité de l'adsorbant ainsi préparé selon le test décrit ci-dessous :

On définit la sélectivité Sél(B/A) d'un adsorbant pour un composé (B) par rapport à un composé (A) comme le rapport des concentrations des composés dans la phase adsorbée divisé par le rapport des concentrations des composés dans la phase non adsorbée à l'équilibre.

L'équation de la sélectivité est la suivante :

$$Sél(B/A) = \frac{(B)z / (A)z}{(B)s / (A)s}$$

où (B)z et (B)s représentent les concentrations de B respectivement dans la zéolite et dans la solution,
où (A)z et (A)s représentent les concentrations de A dans la zéolite et la solution.

EXEMPLE 1 : adsorbant témoin

On agglomère une zéolite NaX industrielle, de rapport Si/Al = 1,25 et de rapport Na/Al = 1, en mélangeant intimement 850 g de poudre de zéolite X, (exprimés en équivalent calciné), 150 g de kaolinite des Charentes (exprimés en équivalent calciné) et 6 g de carboxyméthylcellulose, (adjuvant de rétention destiné à retenir l'eau lors de l'opération d'extrusion) avec la quantité d'eau adéquate pour l'extrusion. L'extrudé est séché, concassé de manière à récupérer des grains dont le diamètre équivalent est égal à 0,7 mm, puis calciné à 550°C sous courant d'azote pendant 2 h. Sa capacité d'adsorption de toluène, déterminée à 25°C et sous une pression partielle de 0,5, est de 20,2 % ; on l'interprète en volume microporeux de 20,2/0,86 =0,235 cm$^3$/g (dans le calcul du volume poreux, on considère que la densité de la phase liquide est identique à la densité du toluène adsorbé, c'est-à-dire 0,86).

Ce granulé est échangé au moyen d'une solution de chlorure de baryum 0,5 M à 95°C en 4 étapes. A chaque étape, le rapport du volume de solution sur la masse de solide est de 20 ml/g et l'échange est poursuivi pendant 4 heures à chaque fois. Entre chaque échange, le solide est lavé plusieurs fois de manière à le débarrasser des excédents de sel. Il est ensuite activé à une température de 250°C pendant 2 h sous courant d'azote.

Le taux d'échange en baryum est de 97 %. La capacité d'adsorption de toluène est de 14,8 %, assimilée à un volume microporeux de 0,17 cm$^3$/g. On mesure également la perte au feu, grandeur importante car elle donne une estimation de l'eau résiduelle présente sur l'adsorbant : on relève ici une perte au feu de 4,5 %. Le volume microporeux mesuré selon la méthode de Dubinin par adsorption d'azote à 77 °K après dégazage sous vide à 300 °C pendant 16 h est de 0,22 cm$^3$/g.

L'application du test de sélectivité décrit plus haut conduit aux résultats suivants :

| Isomères | Sélectivité |
|----------|-------------|
| PX/OX | 2,25 |
| PX/MX | 2,12 |
| PX/EB | 1,77 |
| PX/Tol | 1,52 |

La quantité de paraxylène adsorbé est égale à 0,054 cm$^3$/g.

EXEMPLE 2 : adsorbant selon l'invention

On agglomère une zéolite NaX industrielle, de rapport Si/Al = 1,25 et de rapport Na/Al = 1, en mélangeant intimement 800 g de poudre de zéolite X, (exprimés en équivalent calciné), 150 g de kaolin (exprimés en équivalent calciné), 56 g de silice colloïdale vendue par la société CECA sous la dénomination commerciale Cecasol®30 (et contenant 30 % en poids de SiO$_2$ et 0,5 % de Na$_2$O) et 6 g de carboxyméthylcellulose avec la quantité d'eau adéquate pour l'extrusion. L'extrudé est séché, concassé de manière à récupérer des grains dont le diamètre équivalent est égal à 0,7 mm, puis calciné à 550°C sous courant d'azote pendant 2 h. Sa capacité d'adsorption de toluène, déterminée à 25°C et sous une pression partielle de 0,5, est de 19,8 % ; on l'interprète comme correspondant à un volume microporeux de 0,23 cm$^3$/g à partir de la densité du toluène adsorbé, estimée à partir de celle du toluène liquide.

200 g de granulés ainsi obtenus sont placés dans un réacteur en verre muni d'une double enveloppe régulée à une température de 100 $\pm$ 1 °C puis on ajoute 1,5 l d'une solution aqueuse de soude de concentration 100 g/l et laisse le milieu réactionnel sous agitation pendant 3 h. On procède ensuite au lavage des granulés en 3 opérations successives de lavage à l'eau suivi de la vidange du réacteur. On s'assure de l'efficacité du lavage en mesurant le pH final des eaux de lavage qui doit être compris entre 10 et 10,5.

On détermine la capacité d'adsorption de toluène des granulés ainsi obtenus dans les mêmes conditions que celles décrites à l'exemple 1 : 22,5 %, correspondant à un volume microporeux de 0,26 cm$^3$/g, c'est-à-dire un gain de cristallinité d'environ 13 % par rapport aux granulés de l'exemple 1.

On procède ensuite à un échange baryum dans des conditions opératoires identiques à celles de l'exemple 1 à l'exception de la concentration de la solution de BaCl$_2$ qui est de 0,6 M suivi d'un lavage puis d'un séchage à 80 °C pendant 2 h et enfin d'une activation à 250 °C pendant 2 h sous courant d'azote.

Le taux d'échange en baryum de cet adsorbant est de 97,4 %, sa capacité d'adsorption de toluène est de 16,2 % et sa perte au feu est de 5,2 %. Le volume microporeux mesuré selon la méthode de Dubinin par adsorption d'azote à 77 °K après dégazage sous vide à 300 °C pendant 16 h est de 0,244 cm$^3$/g.

L'application du test de sélectivité décrit plus haut conduit à des résultats semblables à ceux obtenus pour l'adsorbant témoin de l'exemple 1 ; la quantité de paraxylène adsorbé est égale à 0,06 cm$^3$/g.

EXEMPLE 3 (comparatif)

On réalise une unité pilote de chromatographie liquide continue comprenant 24 colonnes en série de 1 m de longueur et 1 cm de diamètre, la circulation entre la 24ème colonne et la 1ère se faisant au moyen d'une pompe de recyclage. On charge chacune de ces colonnes avec l'adsorbant préparé à l'exemple 1 et l'ensemble de l'unité (colonnes + tuyauteries+vannes de distribution) est placé dans une étuve à 150 °C.

Suivant le principe de la chromatographie à contre-courant simulé, on avance de 3 colonnes toutes les 6 min à co-courant de la circulation de liquide, l'injection de solvant, le prélèvement d'extrait, l'injection de la charge et le prélèvement du raffinat : 6 colonnes (soit 2 lits) sont comprises entre l'injection de solvant et le prélèvement d'extrait, 9 colonnes (soit 3 lits) sont comprises entre le prélèvement d'extrait et l'injection de charge, 3 colonnes (1 lit) sont comprises entre l'injection de charge et le prélèvement de raffinat et les 6 dernières colonnes se situent entre le prélèvement de raffinat et l'injection de solvant.

On injecte en continu (exprimés aux conditions ambiantes) 7,3 cm$^3$/min de toluène et 5 cm$^3$/min d'une charge constituée de 21 % en poids de paraxylène, 17 % d'éthylbenzène, 44 % de méta-xylène et 18 % d'ortho-xylène.

On prélève en continu 5,4 cm$^3$/min d'extrait et 6,74 cm$^3$/min de raffinat.

Pendant les 2 premières périodes du cycle, la pompe de recyclage débite (à température ambiante) 38,7 cm$^3$/min ; elle débite 45,5 cm$^3$/min pendant la 3ème période, 40,5 cm$^3$/min pendant les 3 périodes suivantes et 45,9 cm$^3$/min pendant les 2 dernières périodes. Le para-xylène est obtenu avec une pureté de 92,2 % et avec un taux de récupération de 98,1 %. La température est de 150 °C et la pression décroît de 30 à 5 bars. On calcule que la productivité de l'adsorbant est de 0,034 m$^3$ de para-xylène adsorbé par m$^3$ d'adsorbant et par heure.

EXEMPLE 4 (selon l'invention)

On fait maintenant fonctionner l'unité pilote décrite à l'exemple 3 avec l'adsorbant préparé à l'exemple 2. On observe que l'on peut obtenir la même pureté de para-xylène en augmentant le débit de la charge entrant dans l'unité pilote jusqu'à 5,5 cm$^3$/min (soit une augmentation de 10 %).

Pour ce débit de charge, la quantité de désorbant introduite correspond à un débit de 7,92 cm$^3$/min, le temps de permutation est de 5,4 min et la productivité de l'adsorbant est de 0,0374 m$^3$ de para-xylène adsorbé par m$^3$ d'adsorbant et par heure.

**Revendications**

1. Procédé d'obtention d'un adsorbant zéolitique aggloméré consistant en les étapes suivantes :

   a/ on agglomère une zéolite NaX industrielle, de rapport Si/Al = 1,25 et de rapport Na/Al = 1, en mélangeant intimement 800 g de poudre de zéolite X, (exprimés en équivalent calciné), 150 g de kaolin (exprimés en équivalent calciné), 56 g de silice colloïdale (et contenant 30 % en poids de SiO$_2$ et 0,5 % de Na$_2$O) et 6 g de carboxyméthylcellulose avec la quantité d'eau adéquate pour l'extrusion ; l'extrudé est séché, concassé de manière à récupérer des grains dont le diamètre équivalent est égal à 0,7 mm, puis calciné à 550 ° C sous courant d'azote pendant 2 h ;
   b/200 g de granulés ainsi obtenus sont placés dans un réacteur en verre muni d'une double enveloppe régulée à une température de 100± 1 °C puis on ajoute 1,5 l d'une solution aqueuse de soude de concentration 100 g/l et laisse le milieu réactionnel sous agitation pendant 3 h ; on procède ensuite au lavage des granulés en 3 opérations successives de lavage à l'eau suivi de la vidange du réacteur ; on s'assure de l'efficacité du lavage en mesurant le pH final des eaux de lavage qui doit être compris entre 10 et 10,5 ;
   c/ on procède ensuite à un échange baryum au moyen d'une solution de chlorure de baryum 0,6 M à 95°C en 4 étapes ; à chaque étape, le rapport du volume de solution sur la masse de solide est de 20 ml/g et l'échange est poursuivi pendant 4 heures à chaque fois ; suivi d'un lavage puis d'un séchage à 80 °C pendant 2 h entre chaque échange, le solide est lavé plusieurs fois de manière à le débarrasser des excédents de sel ;

d/ il est ensuite activé à une température de 250 °C pendant 2 h sous courant d'azote.

**2.** Procédé de récupération de paraxylène à partir de coupes d'isomères C8 aromatiques en phase liquide, par adsorption du paraxylène au moyen d'un adsorbant susceptible d'être obtenu par le procédé tel que défini à la revendication 1 en présence d'un désorbant.

**3.** Procédé de récupération de paraxylène selon la revendication 2 de type lit mobile simulé.

**4.** Procédé de récupération de paraxylène selon la revendication 3 de type contre-courant simulé.

**5.** Procédé de récupération de paraxylène selon la revendication 3 de type co-courant simulé.

**6.** Procédé de récupération de paraxylène à partir de coupes d'isomères C8 aromatiques en phase gazeuse, par adsorption du paraxylène au moyen d'un adsorbant susceptible d'être obtenu par le procédé tel que défini à la revendication 1 en présence d'un désorbant.

**7.** Procédé de récupération de paraxylène selon l'une quelconque des revendications 3 à 6 dans lequel le désorbant est le toluène ou le paradiéthylbenzène.


**Patentansprüche**

**1.** Verfahren zum Erhalt von einem agglomeriert zeolithisch Adsorptionsmittel, aus den folgenden Schritten :

a) ein industrieller Zeolith NaX mit einem Si / Al-Verhältnis von 1,25 und einem Na / Al-Verhältnis von 1 wird durch inniges Mischen von 800 g Zeolith X-Pulver (ausgedrückt als kalziniertes Äquivalent) und 150 g Kaolin (ausgedrückt als kalziniertes Äquivalent) agglomeriert; 56 g kolloidales Siliciumdioxid (und enthaltend 30 Gew .- % $SiO_2$ und 0,5% $Na_2O$) und 6 g Carboxymethylcellulose mit der für die Extrusion geeigneten Menge Wasser ; das Extrudat wird getrocknet, zerkleinert, um Körner mit einem äquivalenten Durchmesser von 0,7 mm zu gewinnen, und dann 2 h bei 550 °C unter einem Stickstoffstrom calciniert;

b) 200 g so erhaltenes Granulat werden in einen Ummantelung versehenen Glasreaktor gegeben, wobei die doppelter Ummantelung auf eine Temperatur von $100 \pm 1$ °C, 1,5 l einer wässrigen Natriumhydroxidlösung mit einer Konzentration von 100 g/l eingestellt wird ,dann werden zugegeben und das Reaktionsgemisch 3 h rühren gelassen , das Granulat wird anschließend in 3 aufeinanderfolgenden Waschvorgängen mit Wasser gewaschen und anschließend der Reaktor entleert , die Wirksamkeit des Waschens wird durch Messung des End-pH der wässrigen Waschflotten bestätigt, der zwischen 10 und 10,5 liegen muss.

c) dieses Granulat wird mittels einer 0,6 M Bariumchloridlösung bei 95 °C in 4 Stufen ausgetauscht, in jeder Stufe beträgt das Verhältnis von Lösungsvolumen zu Feststoffmasse 20 ml / g und der Austausch wird jeweils 4 Stunden fortgesetzt; gefolgt von Waschen und anschließendem Trocknen bei 80 ° C für 2 Stunden , zwischen jedem Austausch , wird der Feststoff mehrmals gewaschen, um ihn vom überschüssigen Salz zu befreien.

d) schließlich durch Aktivierung bei 250 °C für 2 h unter einem Stickstoffstrom, bestehend wird.

**2.** Verfahren zur Gewinnung von para-Xylol aus aromatischen C8-Isomerenschnitten in der Flüssigphase durch Adsorption von para-Xylol mit Hilfe eines Adsorptionsmittels, das durch das Verfahren nach Ansprüch 1 erhätlich ist, in Gegenwart eines Desorptionsmittels.

**3.** Verfahren zur Gewinnung von para-Xylol nach Anspruch 2 vom Typ Simulated Moving Bed.

**4.** Verfahren zur Gewinnung von para-Xylol nach Anspruch 3 vom Typ simulierter Gegenstrom.

**5.** Verfahren zur Gewinnung von para-Xylol nach Anspruch 3 vom Typ simulierter Gleichstrom.

**6.** Verfahren zur Gewinnung von para-Xylol aus aromatischen C8-Isomerenschnitten in der Gasphase durch Adsorption von para-Xylol mit Hilfe eines Adsorptionsmittels, das durch das Verfahren nach Ansprüch 1 erhätlich ist, in Gegenwart eines Desorptionsmittels.

**7.** Verfahren zur Gewinnung von para-Xylol nach einem des Ansprüche 3 bis 6, bei dem es sich bei dem Desorpti-

# EP 1 864 712 B2

onsmittel um Toluol oder para-Diethylbenzol handelt.

**Claims**

1. Process for producing an agglomerated zeolitic adsorbent consisting of the following stages:

   a) agglomerating an industrial zeolite NaX, with an Si/Al ratio of 1.25 and an Na/Al ratio of 1 by intimately mixing 800 g of zeolite X powder (expressed as calcined equivalent), 150 g of kaolin (expressed as calcined equivalent), 56 g of colloidal silica (and comprising 30% by weight of $SiO_2$ and 0.5% of $Na_2O$), and 6 g of carboxymethylcellulose with the appropriate amount of water for the extrusion ; the extrudate is dried, crushed, so as to recover grains with an equivalent diameter equal to 0.7 mm, and then calcined at 550 °C under a stream of nitrogen for 2 h,
   b) 200 g of granules thus obtained are placed in a glass reactor equipped with a jacket, which jacket is regulated at a temperature of 100±1 °C, then 1.5 l of an aqueous sodium hydroxide solution with a concentration of 100 g/l are then added and the reaction mixture is left stirring for 3 h ;the granules are subsequently washed in 3 successive washing operations with water ; then the reactor is emptied ; the effectiveness of the washing is confirmed by measuring the final pH of the aqueous wash liquors, which must be between 10 and 10.5,
   c) the granules are exchanged by means of a 0.6M barium chloride solution at 95°C in 4 stages; in each stage, the ratio of volume of solution to mass of solid is 20 ml/g and the exchange is continued for 4 hours each time ; followed by washing, then by drying at 80°C. for 2 h, between each exchange, the solid is washed several times, so as to free it from the excess salt;
   d) the granules are then activated at a temperature 250°C for 2 h under a stream of nitrogen.

2. Process for the recovery of para-xylene from C8 aromatic isomer fractions in the liquid phase by adsorption of the para-xylene by means of an adsorbent obtainable by the process according to claim 1 in the presence of a desorbent.

3. Process for the recovery of para-xylene according to claim 2 of simulated moving bed type.

4. Process for the recovery of para-xylene according to claim 3 of simulated countercurrent type.

5. Process for the recovery of para-xylene according to claim 3 of simulated cocurrent type.

6. Process for the recovery of para-xylene from C8 aromatic isomer fractions in the gas phase by adsorption of the para-xylene by means of an adsorbent obtainable by the process according to claim 1 in the presence of a desorbent.

7. Process for the recovery of para-xylene according to any one of claims 3 to 6, in which process the desorbent is toluene or para-diethylbenzene.

## RÉFÉRENCES CITÉES DANS LA DESCRIPTION

**Documents brevets cités dans la description**

- US 3558730 A
- US 3558732 A
- US 3626020 A
- US 3663638 A
- US 3878127 A
- US 2985589 A
- EP 115631 A
- EP 115068 A
- EP 137063 A
- US 4642397 A
- US 4940548 A
- US 4633018 A
- US 5149887 A
- US 3119660 A
- US 4818508 A
- JP 05163015 B
- JP 5163015 A
- US 5284992 A
- US 5629467 A
- US 4402832 A
- US 4498991 A

**Littérature non-brevet citée dans la description**

- **D.W. BRECK.** ZEOLITE MOLECULAR SIEVES. John Wiley and Sons
- **BRECK.** *ZEOLITE MOLECULAR SIEVES,* 320